(19) Europäisches Patentamt
European Patent Office
Office européen des brevets

(11) Publication number: 0 277 641 A2

# (12) EUROPEAN PATENT APPLICATION

(21) Application number: 88101489.8

(51) Int. Cl.4: A61K 7/48 , A61K 7/04

(22) Date of filing: 02.02.88

(30) Priority: 05.02.87 JP 25436/87

(43) Date of publication of application: 10.08.88 Bulletin 88/32

(84) Designated Contracting States: AT CH DE ES FR GB IT LI NL

(71) Applicant: Kao Corporation
1-14-10, Nihonbashi Kayaba-cho
Chuo-ku Tokyo(JP)

(72) Inventor: Tokimitsu, Ichiro
7-23-616, Shinogawa-machi
Shinjuku-ku Tokyo(JP)
Inventor: Asahi, Masahiko
4-34-2, Otsuka
Bunkyo-ku Tokyo(JP)
Inventor: Suzuki, Toshiyuki
1-5-8, Motokitakata
Ichikawa-shi Chiba-ken(JP)

(74) Representative: Wächtershäuser, Günter, Dr.
Tal 29
D-8000 München 2(DE)

## (54) Cosmetics.

(57) A cosmetic comprising as its essential components, (A) a horny layer lipid or its analogous substance and (B) a liquid di-fatty acid glycerol ester, provides a high degree of satisfaction on use, and exhibits excellent moisturizing and moisture-retaining effects. Horny layer lipids, both derived from living organisms and artificially synthesized, are used. Even better effect of the cosmetic can be obtained by the further addition of a low-molecular weight humectant component.

EP 0 277 641 A2

# COSMETICS

## BACKGROUND OF THE INVENTION:

### Field of the Invention

This invention relates to a cosmetic, and, more particularly, to a cosmetic which has a long-lasting, high moisturizing effect and imparts satisfaction on use.

### Description of the Background:

Conventionally, low-molecular weight hydrophilic substances such as glycerol, sorbitol, 1,3-butylene glycol, and the like, natural moisturizing factors (NMF) such as amino acid, sodium pyrrolidone carboxylate, urea, and the like, and high-molecular weight extracts from living bodies such as mucopolysaccharide, collagen, and the like have been used as humectants for cosmetics. The moisture-retaining abilities of these humectants depend on their hydrophillic properties. These substances provide only temporary moisturizing effects, however, with no fundamental improvement in the moisture-retaining ability of the human horny layer. Thus, the substances cannot give long-lasting moisturizing effects.

The present inventors gave attention to the fact that horny layer lipids play an important roll in the moisture-retention of horny layers, and found that if these lipids or substances with structures similar to such lipids (such lipids and substances are herein from time to time collectively called "lipids"), ceramides or their analogous compounds in particular, are formulated in a cosmetic, they could provide fundamental improvements in the moisture-retaining ability of human horny layers.

However, merely formulating these lipids in a cosmetic does not always bring about satisfactory results in terms of the persistency of long-lasting effects of its moisture retention. In addition, a problem continues with the consistency of these lipids and oil components generally used in cosmetics. This causes unsatisfactory results in the feelings on use of the cosmetics in which these lipids are formulated, such as insufficient extendability on the skin surface or inadequate fitness to the skin when applied.

In view of this situation, the present inventors conducted extensive and earnest studies to develop a cosmetic which possesses a long-lasting, high moisturizing effect, and provides complete satisfaction on use. As a result, the inventors found that by using a horny layer lipid and/or a substance with a similar structure therewith, along with a liquid di-fatty acid glycerol ester, a cosmetic can be obtained which produce an outstanding moisturizing effect lasting for a long period of time, and provides satisfaction to users when applied. The inventors also found that when a water-soluble humectant substance is used in conjunction with these compounds, a futher promotion of the moisturizing effect can be realized. Such findings have led to the completion of this invention.

## SUMMARY OF THE INVENTION

Accordingly, an object of this invention is to provide a cosmetic comprising (A) 0.1 - 50% by weight of a horny layer lipid and/or a substance with a structure similar thereto, and (B) 0.1 - 50% by weight of a liquid di-fatty acid glycerol ester.

Another object of this invention is to provide a cosmetic comprising, in addition to the above components (A) and (B), a low-molecular weight humectant component.

Still other object of this invention is to provide a cosmetic having excellent moisturizing and moisture-retaining effects and providing a high degree of satisfaction on use.

Other objects, features, and advantages of this invention will appear more fully from the following description.

DETAILED DESCRIPTION OF THE INVENTION AND PREFERRED EMBODIMENTS

Horny layer lipids used as component (A) of this invention are lipids normally existing in intercellular spaces of a living body, and include, for example, a ceramide, glycoceramide, cholesterol, saturated or unsaturated, linear or branched fatty acid having 12 - 36, preferably 14 - 28, carbon atoms, which may contain one or more hydroxyl groups as substituents (hydroxyl fatty acid), fatty acid ester of cholesterol, in which fatty acid is saturated or unsaturated, linear or branched and having 12 -36, preferably 14 - 28, carbon atoms, with or without one or more hydroxyl groups as substituents, and the like. These lipids are described, for example, by G. M. Gray and H. J. Yardley [J. Lipid Reasearch, 16, 434 - 440 (1975)]. Among these, desirable horny lipids are ceramides, glycoceramides, and cholesterol esters. Ceramides and glyciceramides may be either those extracted from naturally occurring substances such as brain tissues of horny layer of mammals, or the like, or those artificially synthesized.

As examples of substances having a structure similar to a horny layer lipid, the following compounds may be given:

A substance with a structure similar to a ceramide may be a lipid derivative which has two long-chained hydrocarbon groups with an OH group and amide group between them, and whose molecule is capable of assuming its configulation on a single plane. Typical examples are the compounds represented by the following formula (I):

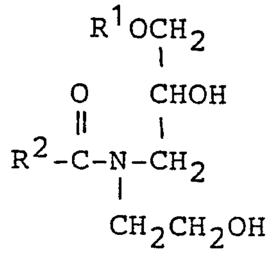

(I)

in which $R^1$ represents a linear or branched, saturated or unsaturated hydrocarbon group having 10 - 26 carbon atoms and $R^2$ represents a linear or branched, saturated or unsaturated hydrocarbon group having 9 - 25 carbon atoms. A cholesterol ester may be a cholesterol ester of a fatty acid with a linear or branched, saturated or unsaturated hydrocarbon group having 8 - 20 carbon atoms. Particularly desirable cholesterol esters are cholesterol esters of a monomethyl-branched fatty acid.

These horny layer lipids and substances with structures similar to those of horny layer lipids may be used either independently or mixed with one or more other compounds. The amount to be formulated depends on the cosmetic form, but generally a desirable range is 0.1 to 50% by weight (hereinafter designated simply as "%") of the total weight of the composition.

Liquid di-fatty acid glycerol esters to be used as component (B) of this invention are those having melting points of not more than 30°C. They can be obtained by first preparing crude di-fatty acid glycerol esters having a diester content of not less than 50% by means of conventional processes, for example, by esterification of a glycerol and a fatty acid, glycerolysis of glycerol and an oil or fat (either hydrogenated or non-hydrogenated) originating from animals or plants, or glycerolysis of a higher fatty acid-lower alcohol ester and glycerol, and then by purification of di-fatty acid glycerol esters by means of molecular distillation, solvent extraction, or the like. Among such liquid di-fatty acid glycerol esters, particularly preferable compounds are di-fatty acid glycerol esters of one or more fatty acids selected from 2-ethyl hexanoic acid, myristic acid, oleic acid, iso-stearic acid, or the like. The amount of liquid di-fatty acid glycerol ester to be formulated may be 0.5 to 50% of the total amount of the cosmetic composition. A preferable amount based on the amount of horny layer lipids or substances analogous therewith is 0.1 to 20 times, and particularly preferably is 0.1 to 10 times, the amount of the latter compounds.

Cosmetics prepared according to the present invention can provid a more excellent moisturizing effect by formulating a low-molecular weight humectant as component (C). Such a humectant may be, for example, a polyol, including glycerol, 1,3-butylene glycol, dipropylene glycol, propylene glycol, ethylene glycol, sorbitol, pentaerythritol, or the like, natural moisturizing factors, including amino acid, sodium pyrrolidone carboxylate, urea, or the like, or a glycolipid typified by polyoxypropylene[(2'-O-β-glucopyranosyl-β-glucopyranosyl)oxy] fatty acid ester (hereinafter abbreviated as PSL). Among these, particularly preferable compounds are glycerol, 1,3-butylene glycol, dipropylene glycol, and PSL. The amount of low-molecular weight humectants to be formulated may be 1 to 50% of the total amount of the cosmetic composition. A preferable amount based on the amount of liquid di-fatty acid glycerol esters is 0.5

to 50 times the amount of the latter.

In addition to these components, glyceryl ethers may be formulated as absorption promoters of horny layer lipids or substances analogous thereto. Preferable are glyceryl ethers represented by following formula (II) are preferable:

$$R\text{-}OCH_2\underset{\displaystyle OH}{\underset{|}{CH}}\text{-}CH_2OH \qquad (II)$$

in which R represents an alkyl group having 8 - 24 carbon atoms. Particularly preferable glyceryl ethers are those of formula (II) wherein R is a group represented by formula (III):

$$CH_3 \text{—}(CH_2)_p\text{—}\underset{\displaystyle CH_3}{\underset{|}{CH}}\text{-}(CH_2)_q\text{—} \qquad (III)$$

in which p is an integer of 4 to 10 and q is an integer of 5 to 11, provided that p and q satisfy the equation $p + q = 11 - 17$, and further that the distributions of p and q peak at $p = 7$ and $q = 8$. The amount of these compounds to be formulated is preferably 0.01 to 20%, and particularly preferably is 0.1 to 5%, of the total amount of the cosmetic composition.

Beside the above-mentioned essential components, the costmetics according to this invention may be compounded with various ingredients commonly used in cosmetics inasmuch as the same does not impair the effect of this invention. Such other ingredients include cosmetic oils, surface active agents, ultraviolet absorbers, chelating agents, pH adjusting agents, antiseptics, vehicles, coloring agents, perfumes, and the like.

Cosmetics of this invention can be made into various forms by means of conventional methods, including basic cosmetics such as oil-in-water or water-in-oil-type emulsion cosmetics and oily type cosmetics, make-up cosmetics such as lipsticks and foundations, skin cleansers, hair cosmetics such as hair tonics, hair-set lotions, hair creams, and the like.

Although it has not yet been completely elucidated, the mechanism by which the outstanding effect possible through this invention can be brought about is presumed to be as follows. Penetration of a lipid of component (A) and a low-molecular weight humectant substance of component (C) into horny layers is promoted by a liquid di-fatty acid glycerol ester of component (B). Such penetration of components (A) and (C) into horny layers improves their moisture-retaining ability of the layers, and, at the same time, contributes to a long-lasting moisturizing effect at such layers. In addition, the amphoteric characteristic of the liquid di-fatty acid glycerol ester of component (B) promotes compatibility of lipids of component (A) and commonly used cosmetic oils, which, in conjunction with excellent characteristics of the liquid di-fatty acid glycerol ester, provides a cosmetic delivering greater satisfaction on use.

Other features of the invention will become apparent in the course of the following description of the exemplary embodiments which are given for illustration of the invention and are not intended to be limiting thereof.

## EXAMPLES

### Example 1

Creams of formulations shown in Table 1 were prepared, and subjected to evaluation by means of actual application and to measurement of moisture retention effects.

Table 1

(wt. %)

| Commponents | Comparativer Compositions No. 1 | No. 2 | No. 3 | No.4 | No.5 | Inventive Compositions No. 1 | No. 2 | No. 3 |
|---|---|---|---|---|---|---|---|---|
| (1) petrolatum | 6.0 | 6.0 | 6.0 | 6.0 | 6.0 | 6.0 | 6.0 | 6.0 |
| (2) cetanol | 5.0 | 5.0 | 5.0 | 5.0 | 5.0 | 5.0 | 5.0 | 5.0 |
| (3) liquid paraffine | 25.0 | - | - | - | - | - | - | - |
| (4) tri(2-ethylhexanoic acid) glycerol ester | - | 25.0 | 25.0 | - | - | - | - | - |
| (5) di(2-ethylhexanoiic acid) glycerol ester | - | - | - | 25.0 | - | 25.0 | 25.0 | 25.0 |
| (6) lipid * | 5.0 | 5.0 | 5.0 | - | 5.0 | 5.0 | 5.0 | 5.0 |
| (7) mono-fatty acid glycerol ** | 2.0 | 2.0 | 2.0 | 2.0 | 2.0 | 2.0 | 2.0 | 2.0 |
| (8) POE(20) sorbitan monolauric acid ester | 2.0 | 2.0 | 2.0 | 2.0 | 2.0 | 2.0 | 2.0 | 2.0 |
| (9) glyceryl ether *** | - | - | - | - | - | - | - | 2.0 |
| (10) glycerol | - | - | 15.0 | - | - | - | 15.0 | 15.0 |
| (11) ethyl paraben | 0.1 | 0.1 | 0.1 | 0.1 | 0.1 | 0.1 | 0.1 | 0.1 |
| (12) methyl paraben | 0.2 | 0.2 | 0.2 | 0.2 | 0.2 | 0.2 | 0.2 | 0.2 |
| (13) perfume | 0.1 | 0.1 | 0.1 | 0.1 | 0.1 | 0.1 | 0.1 | 0.1 |
| (14) purified water | balance | balance | balance | balance | balance | balance | balance | balance |

* : lipid in the formula (I) in which $R^1 = C_{16}H_{33}$ and $R^2 = C_{15}H_{31}$

** : monolauryl glycerol

*** : compound of the formula (II) in which R is the formula (III)

<Preparation>

Oil phase components (1) to (9) of Table 1 were mixed and heated to melt, and maintained at a temperature of 70°C. To this mixture were slowly added water phase components (10) to (14), and the resulting mixture was emulsified by a mixer. The emulsion thus obtained was cooled by a heat exchanger to a final temperature of 30°C, and poured into containers to give inventive compositions Nos. 1, 2, and 3. Comparative compositions Nos. 1 to 5 were prepared in the same manner.

<Test Methods>

(1) Evaluation of compositions by actual application.

Evaluation of the compositions prepared as above was carried out by 10 expert panelists. After applying the creams to their skin, each panelist was asked to give their feelings as to (i) fitness to the skin, (ii) dampishness, (iii) stickilessness, (iv) dampishness of their skin at 3 hours after application and (v) overall rating. The creams were then rated according to the panelists' remarks as follows:

AAA: at least 8 out of 10 panelists expressed their complete satisfaction.
BBB: at least 6 out of 10 panelists expressed their complete satisfaction.
CCC: at least 4 out of 10 panelists expressed their complete satisfaction.
DDD: less than 4 out of 10 panelists expressed their complete satisfaction.

(2) Measurements of moisturizing effect and moisture-retaining effect

A prescribed amount of each sample was applied to axillary side of the arm of each subject, left for 3 hours, and then washed off with hot water. The subjects stayed in a room with a constant temperature of 20°C and humidity of 50%. After 30 minutes, the moisture content of the horny layer was measured by an impedance meter (manufactured by IBS Co.) to determine the value of the moisturizing effect. Three hours later, washing with hot water was repeated, and the subjects again entered the room with a constant temperature of 20°C and humidity of 50%. At 30 minutes thereafter measurement of the moisture content in the horny layer was again conducted by the impedance meter. The value thus obtained indicates the moisture-retaining effect. In Table 2, both moisturizing effect and moisture-retaining effect was indicated in means values (N = 5).

Table 2

(wt. %)

| Actual Application Test | Comparativer Compositions | | | | | Inventive Compositions | | |
|---|---|---|---|---|---|---|---|---|
| | No. 1 | No. 2 | No. 3 | No.4 | No.5 | No. 1 | No. 2 | No. 3 |
| (1) Fitness to the skin | CCC | BBB | BBB | BBB | CCC | AAA | AAA | AAA |
| (2) Dampishness | CCC | CCC | BBB | CCC | BBB | BBB | AAA | AAA |
| (3) Stikilessness | CCC | CCC | DDD | BBB | CCC | AAA | BBB | BBB |
| (4) Extendability on application | CCC | CCC | CCC | CCC | DDD | AAA | AAA | AAA |
| (5) Overall rating | CCC | CCC | BBB | BBB | BBB | AAA | AAA | AAA |
| Moisturizing effect * | 71 | 78 | 112 | 83 | 131 | 146 | 163 | 190 |
| Moisture retaining effect * | 35 | 41 | 53 | 50 | 83 | 95 | 101 | 114 |

* unit:

As is evident from Table 2, as compared with cosmetics in which either a di-fatty acid glyceryl ester or horny layer lipid is independently formulated, the cosmetics of this invention containing both of these ingredients provide better fitness to the skin and superiorority in their moisturizing and moisture-retention effects. Thus, it is apparent that these ccmponents exert a synergistic effect in the cosmetic compositions.

Example 2 Cream

| | |
|---|---|
| Oil phase components: | |
| stearic acid | 2.0% |
| cetanol | 3.0 |
| 2-ethylhexanoic acid-myristic acid diglyceride | 1.0 |
| lipid [having $R^1$ of $C_{16}H_{33}$ and $R^2$ of $C_{15}H_{31}$ in formula (I)] | 10.0 |
| monolauryl glycerol | 2.0 |
| POE(20) sorbitan-monolauric acid ester | 2.0 |
| glyceryl ether [of formula (II) having R of formula (III)] | 2.0 |
| Water phase components: | |
| dipropylene glycol | 10.0 |
| 1,3-butylene glycol | 5.0 |
| ethyl paraben | 0.1 |
| methyl paraben | 0.2 |
| perfume | 0.1 |
| purified water | balance |
| | 100.0% |

The oil phase components were mixed and heated to dissolve, and maintained at a temperature of 70°C. To this mixture were slowly added the water phase components at 70°C, and the resulting mixture was emulsified by a mixer. The emulsion thus obtained was cooled by a heat exchanger to a final temperature of 30°C, and poured into containers to give a cream.

Example 3 Milky lotion

| | |
|---|---|
| Oil phase components: | |
| cetanol | 0.5% |
| petrolatum | 1.0 |
| Di-oleic acid glycerol ester | 10.0 |
| lipid [ceramide (mnufactured by Sedary Research Laboratories, Inc.] | 1.0 |
| POE(10) monooleic acid ester | 2.0 |
| stearic acid | 2.0 |
| Water phase components: | |
| PSL | 2.0 |
| propylene glycol | 3.0 |
| triethanolamine | 1.0 |
| ethyl paraben | 0.1 |
| methyl paraben | 0.2 |
| perfume | 0.1 |
| purified water | balance |
| | 100.0% |

The oil phase components were mixed and heated to dissolve, and maintained at a temperature of 70°C. To this mixture were slowly added water phase components at 70°C, and the resulting mixture was emulsified by a mixer. The emulsion thus obtained was cooled by a heat exchanger to a final temperature of 30°C, and poured into containers to give a milky lotion.

Example 4 Milky foundation

| | |
|---|---|
| Oil phase components: | |
| stearic acid | 5.0% |
| ceto-stearyl alcohol | 1.0 |
| 2-ethylhexanoic acid-oleic acid diglyceride | 6.0 |
| lipid (iso-stearic acid cholesterol ester) | 6.0 |
| monolauryl glycerol | 2.0 |
| monolauric acid propylene glycol ester | 3.0 |
| Water phase components: | |
| glycerol | 10.0 |
| triethanolamine | 1.2 |
| ethyl paraben | 0.1 |
| methyl paraben | 0.1 |
| perfume | 0.1 |
| purified water | balance |
| Powdery components: | |
| titanium oxide | 8.0 |
| talc | 4.0 |
| iron oxide | 0.5 |
| | 100.0% |

The oil phase components were mixed and heated to dissolve, and maintained at a temperature of 70²-[x² ¥ C. The powdery components were added to and dispersed in the water phase components, and heated to 70°C. The water phase thus prepared was added to the oil phase, and emulsified by a mixer. Emulsion thus obtained was cooled by a heat exchanger to a final temperature of 30°C, ar.d poured into containers to give a milky foundation.

Example 5 Lipstick

Formulation:

| | |
|---|---|
| microcrystalline wax | 6.0% |
| canderilla wax | 3.0 |
| di-stearyl acid glycerol ester | 15.0 |
| lipid [Sereproside (mnufactured by Sedary Research Laboratories, Inc.] | 2.0 |
| jojoba oil | 6.0 |
| olive oil | balance |
| lanolin | 10.0 |
| pigment | 7.0 |
| perfume | 0.1 |
| | 100.0% |

Microcrystalline wax, canderilla wax, di-stearyl acid glycerol ester, lipid, jojoba oil, olive oil, and lanolin were mixed and heated to 90 - 100°C to dissolve. To this mixture pigment was added and agitated thoroughly to disperse it in the mixture, which was then deaerated, and the perfume was added. This was then charged into lipstick mold, allowed to cool to 15 - 20°C, and released from the mold to obtain the lipstick.

The Cosmetics according to the present invention prepared in Examples 2 to 5 all produced a high degree of satisfaction on use, and exhibited excellent moisturizing and moisture-retaining effects.

Obviously, numerous modifications and variations of the present invention are possible in light of the above teachings. It is therefore to be understood that the scope of the appended claims, the invention may be practiced otherwise than as specifically described herein.

## Claims

1. A cosmetic comprising:
   (A) 0.1 - 50% by weight of a horny layer lipid and/or a substance with a structure similar thereto, and
   (B) 0.1 - 50% by weight of a liquid di-fatty acid glycerol ester.
2. A cosmetic according to Claim 1, wherein said horny layer lipid is a member selected from the group consisting of a ceramide, glycoceramide, cholesterol, higher fatty acid, and fatty acid ester of cholesterol.
3. A cosmetic according to Claim 1, wherein said substance with a structure similar to a horny layer lipid is a compound represented by the following formula (I):

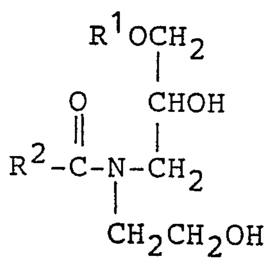

(I)

in which $R^1$ represents a linear or branched, saturated or unsaturated hydrocarbon group having 10 - 26 carbon atoms and $R^2$ represents a linear or branched, saturated or unsaturated hydrocarbon group having 9 - 25 carbon atoms.

4. A cosmetic according to Claims 1, 2, or 3, further comprising 1 - 50% by weight of a low-molecular weight humectant component.